# EUROPEAN PATENT SPECIFICATION

(11) **EP 1 740 180 B1**
(45) Date of publication and mention of the grant of the patent: **21.07.2010**
(21) Application number: 05735420.1
(22) Date of filing: 18.04.2005
(51) Int. Cl.: A61K 31/496, A61P 15/00

(54) **USE OF FLIBANSERIN IN THE TREATMENT OF PREMENSTRUAL DISORDERS**
VERWENDUNG VON FLIBANSERIN BEI DER BEHANDLUNG VON PRÄMENSTRUELLEN STÖRUNGEN
UTILISATION DE FLIBANSERINE DANS LE TRAITEMENT DES TROUBLES PREMENSTRUELS

(30) Priority: 22.04.2004 US 564660 P
(43) Date of publication of application: 10.01.2007
(73) Proprietor: Boehringer Ingelheim Pharmaceuticals, Inc., Ridgefield Connecticut 06877 (US); Boehringer Ingelheim Pharma GmbH & Co. KG, 55216 Ingelheim (DE)
(72) Inventor: PYKE, Robert, Ridgefield, Connecticut 06877 (US)
(74) Representative: Hammann, Heinz
(86) International application number: PCT/EP2005/004086
(87) International publication number: WO 2005/102343

(56) References cited:
- EP-A- 0 526 434
- EP-A- 0 526 434
- WO-A-01/21593
- WO-A-01/21593
- WO-A-03/035072
- WO-A-03/035072
- WO-A-03/097058
- WO-A-2005/007166
- US-A1- 2003 104 980
- STEINER M: "Recognition of premenstrual dysphoric disorder and its treatment" LANCET THE, LANCET LIMITED. LONDON, GB, vol. 356, no. 9236, 30 September 2000 (2000-09-30), pages 1126-1127, XP004815104 ISSN: 0140-6736
- DARLINGTON C: "FLIBANSERIN BOEHRINGER INGELHEIM CORP" CURRENT OPINION IN CPNS INVESTIGATIONAL DRUGS, PHARMA PRESS, LONDON,, GB, vol. 1, no. 4, 1999, pages 510-513, XP001119016 ISSN: 1464-844X
- DIMMOCK P W ET AL: "Efficacy of selective serotonin-reuptake inhibitors in premenstrual syndrome: a systematic review" LANCET THE, LANCET LIMITED. LONDON, GB, vol. 356, no. 9236, 30 September 2000 (2000-09-30), pages 1131-1136, XP004815109 ISSN: 0140-6736
- STEINER M: "Recognition of premenstrual dysphoric disorder and its treatment" LANCET THE, LANCET LIMITED. LONDON, GB, vol. 356, no. 9236, 30 September 2000 (2000-09-30), pages 1126-1127, XP004815104 ISSN: 0140-6736
- DARLINGTON C: "FLIBANSERIN BOEHRINGER INGELHEIM CORP" CURRENT OPINION IN CPNS INVESTIGATIONAL DRUGS, PHARMA PRESS, LONDON,, GB, vol. 1, no. 4, 1999, pages 510-513, XP001119016 ISSN: 1464-844X
- DIMMOCK P W ET AL: "Efficacy of selective serotonin-reuptake inhibitors in premenstrual syndrome: a systematic review" LANCET THE, LANCET LIMITED. LONDON, GB, vol. 356, no. 9236, 30 September 2000 (2000-09-30), pages 1131-1136, XP004815109 ISSN: 0140-6736

## Description

The invention relates to a method for the treatment of premenstrual disorders comprising the administration of a therpeutically effective amount of flibanserin.

### Description of the invention

The compound 1-[2-(4-(3-trifluoromethyl-phenyl)piperazin-1-yl)ethly]-2,3-dihydro-1H-benzimidazol-2-one (flibanserin) is disclosed in form of its hydrochloride in European Patent Application EP-A-526434 and has the following chemical structure:

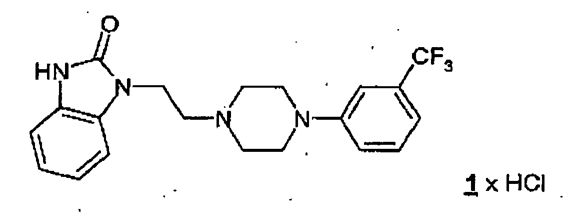

Flibanserin shows affinity for the 5-HT_{1A} and 5-HT₂-receptor, It is therefore a promising therapeutic agent for the treatment of a variety of diseases, for instance depression, schizophrenia, and anxiety.

In studies of female patients suffering from sexual dysfunction it has been found that flibanserin optionally in form of the pharmacologically acceptable acid addition salts thereof proved to be effective in the treatment of premenstrual disorders. Accordingly, the instant invention relates to a method for the treatment of premenstrual disorders comprising the administration of a therapeutically effective amount of flibanserin, optionally in form of the pharmacologically acceptable acid addition salts thereof.

In a preferred embodiment the invention relates to a method for the treatment of premenstrual disorders selected from the group consisting of premenstrual dysphoria, premenstrual syndrome, premenstrual dysphoric disorder, comprising the administration of a therapeutically effective amount of flibanserin, optionally in form of the pharmacologically acceptable acid addition salts thereof.

Further the invention discloses a method for the treatment of sexual aversion disorder in females comprising the administration of a therapeutically effective amount of flibanserin, optionally in form of the pharmacologically acceptable acid addition salts thereof.

Further the invention discloses a method for the treatment of sexual arousal disorder in females comprising the administration of a therapeutically effective amount of flibanserin, optionally in form of the pharmacologically acceptable acid addition salts thereof.

Further the invention discloses a method for the treatment of orgasmic disorder in females comprising the administration of a therapeutically effective amount of flibanserin, optionally in form of the pharmacologically acceptable acid addition salts thereof.

Further the invention discloses a method for the treatment of sexual pain disorders in females comprising the administration of a therapeutically effective amount of flibanserin, optionally in form of the pharmacologically acceptable acid addition salts thereof.

In a particular the invention discloses a method for the treatment sexual pain disorders selected from the group consisting of dyspareunia, vaginismus, noncoital sexual pain disorder, sexual dysfunction due to a general medical condition and substance-induced sexual dysfunction comprising the administration of a therapeutically effective amount of flibanserin, optionally in form of the pharmacologically acceptable acid addition salts thereof.

Another embodiment of the invention relates to the use of flibanserin, optionally in form of the pharmacologically acceptable acid addition salts thereof for the preparation of a medicament for the treatment of the aforementioned disorders.

The beneficial effects of flibanserin can be observed regardless of whether the disturbance existed lifelong or was acquired, and independent of etiologic origin (organic- both, physically and drug induced-, psychogen, a combination of organic-both, physically and drug induced-, and psychogen, or unknown).

Flibanserin can optionally used in form of its pharmaceutically acceptable acid addition salts. Suitable acid addition salts include for example those of the acids selected from, succinic acid, hydrobromic acid, acetic acid, fumaric acid, maleic acid, methanesulphonic acid, lactic acid, phosphoric acid, hydrochloric acid, sulphuric acid, tartaric acid and citric acid. Mixtures of the abovementioned acid addition salts may also be used, From the aforementioned acid addition salts the hydrochloride and the hydrobromide, particularily the hydrochloride, are preferred.

Flibanserin, optionally used in form of its pharmaceutically acceptable acid addition salts, may be incorporated into the conventional pharmaceutical preparation in solid, liquid or spray form. The composition may, for example, be presented in a form suitable for oral, rectal, parenteral administration or for nasal inhalation: preferred forms includes for example, capsules, tablets, coated tablets, ampoules, suppositories and nasal spray.

The active ingredient may be incorporated in excipients or carriers conventionally used in pharmaceutical compositions such as, for example, talc, arabic gum, lactose, gelatine, magnesium stearate, corn starch, acqueous or non acqueous vehicles, polyvynil pyrrolidone, semisynthetic glicerides of fatty acids, benzalconium chloride, sodium phosphate , EDTA, polysorbate 80. The compositions are advantageously formulated in dosage units, each dosage unit being adapted to supply a single dose of the active ingredient. The dosis range applicable per day is between 0.1 to 400, preferably between 1.0 to 300, more preferably between 2 to 200 mg.

Each dosage unit may conveniently contain from 0,01 mg to 100 mg, preferably from 0,1 to 50 mg.

Suitable tablets may be obtained, for example, by mixing the active substance(s) with known excipients, for example inert diluents such as calcium carbonate, calcium phosphate or lactose, disintegrants such as corn starch or alginic acid, binders such as starch or gelatine, lubricants such as magnesium stearate or talc and/or agents for delaying release, such as carboxymethyl cellulose, cellulose acetate phthalate, or polyvinyl acetate. The tablets may also comprise several layers.

Coated tablets may be prepared accordingly by coating cores produced analogously to the tablets with substances normally used for tablet coatings, for example collidone or shellac, gum arabic, talc, titanium dioxide or sugar. To achieve delayed release or prevent incompatibilities the core may also consist of a number of layers. Similarly the tablet coating may consist of a number or layers to achieve delayed release, possibly using the excipients mentioned above for the tablets.

Syrups or elixirs containing the active substances or combinations thereof according to the invention may additionally contain a sweetener such as saccharine, cyclamate, glycerol or sugar and a flavour enhancer, e.g of a flavouring such as vanilline or orange extract. They may also contain suspension adjuvants or thickeners such as sodium carboxymethyl cellulose, wetting agents such as, for example, condensation products of fatty alcohols with ethylene oxide, or preservatives such as p-hydroxybenzoates.

Solutions for injection are prepared in the usual way, e.g of. with the addition of preservatives such as p-hydroxybenzoates, or stabilisers such as alkali metal salts of ethylenediamine tetraacetic acid, and transferred into injection vials or ampoules.

Capsules containing one or more active substances or combinations of active substances may for example be prepared by mixing the active substances with inert carriers such as lactose or sorbitol and packing them into gelatine capsules.

Suitable suppositories may be made for example by mixing with carriers provided for this purpose, such as neutral fats or polyethyleneglycol or the derivatives thereof.

The Examples which follow illustrate the present invention :

### Examples of pharmaceutical formulations

### A)

| Tablets | per tablet |
|---|---|
| flibanserin hydrochloride | 100 mg |
| lactose | 240 mg |
| corn starch | 340 mg |
| polyvinylpyrrolidone | 45 mg |
| magnesium stearate | 15 mg |
| | 740 mg |

The finely ground active substance, lactose and some of the corn starch are mixed together. The mixture is screened, then moistened with a solution of polyvinylpyrrolidone in water, kneaded, wet-granulated and dried. The granules, the remaining corn starch and the magnesium stearate are screened and mixed together. The mixture is compressed to produce tablets of suitable shape and size.

### B)

| Tablets | per tablet |
|---|---|
| flibanserin hydrochloride | 80 mg |
| corn starch | 190 mg |
| lactose | 55 mg |
| microcrystalline cellulose | 35 mg |
| polyvinylpyrrolidone | 15 mg |
| sodium-carboxymethyl starch | 23 mg |
| magnesium stearate | 2 mg |
| | 400 mg |

The finely ground active substance, some of the corn starch, lactose, microcrystalline cellulose and polyvinylpyrrolidone are mixed together, the mixture is screened and worked with the remaining corn starch and water to form a granulate which is dried and screened. The sodium-carboxymethyl starch and the magnesium stearate are added and mixed in and the mixture is compressed to form tablets of a suitable size:

### C)

| Coated tablets | per coated tablet |
|---|---|
| | |
| flibanserin hydrochloride | 5 mg |
| corn starch | 41.5 mg |
| lactose | 30 mg |
| polyvinylpyrrolidone | 3 mg |
| magnesium stearate | 0.5 mg |
| | 80 mg |

The active substance, corn starch, lactose and polyvinylpyrrolidone are thoroughly mixed and moistened with water. The moist mass is pushed through a screen with a 1 mm mesh size, dried at about 45°C and the granules are then passed through the same screen. After the magnesium stearate has been mixed in, convex tablet cores with a diameter of 6 mm are compressed in a tablet-making machine. The tablet cores thus produced are coated in known manner with a covering consisting essentially of sugar and talc. The finished coated tablets are polished with wax.

### D)

| Capsules | per capsule |
|---|---|
| | |
| flibanserin hydrochloride | 1 50 mg |
| Corn starch | 268.5 mg |
| Magnesium stearate | 1.5 mg |
| | 420 mg |

The substance and corn starch are mixed and moistened with water. The moist mass is screened and dried. The dry granules are screened and mixed with magnesium stearate. The finished mixture is packed into size 1 hard gelatine capsules.

### E) Ampoule solution

| | |
|---|---|
| flibanserin hydrochloride | 50 mg |
| sodium chloride | 50 mg |
| water for inj. | 5 ml |

The active substance is dissolved in water at its own pH or optionally at pH 5.5 to 6.5 and sodium chloride is added to make it isotonic. The solution obtained is filtered free from pyrogens and the filtrate is transferred under aseptic conditions into ampoule which are then sterilised and sealed by fusion.

### F) Suppositories

| | |
|---|---|
| flibanserin hydrochloride | 50 mg |
| solid fat | 1650 mg |
| | 1700 mg |

The hard fat is melted. At 40°C the ground active substance is homogeneously dispersed. It is cooled to 38°C and poured into slightly chilled suppository moulds.

In a particular preferred embodiment of the instsnt invention, flibanserin is administered in form of specific film coated tablets. Examples of these preferred formulations are listed below. The film coated tablets listed below can be manufactured according to procedures known in the art (see hereto WO 03/097058).

### G) Film coated tablet

### Core

| **Constituents** | **mg/tablet** |
|---|---|
| Flibanserin | 25.000 |
| Lactose monohydrate | 71.720 |
| Microcrystalline cellulose | 23.905 |
| HPMC (Methocel E5) | 1.250 |
| Carboxymethylcellulose sodium | 2.500 |
| Magnesium stearate | 0.625 |

### Coating

| **Constituents** | **mg/ tablet** |
|---|---|
| HPMC (Methocel E5) | 1.440 |
| Polyethylene Glycol 6000 | 0.420 |
| Titanium dioxide | 0.600 |
| Talc | 0.514 |
| Iron oxide red | 0.026 |
| | |
| **Total Film coated tablet** | **128.000** |

### H) Film coated tablet

### Core

| **Constituents** | **mg/tablet** |
|---|---|
| Flibanserin | 50.000 |
| Lactose monohydrate | 143.440 |
| Microcrystalline cellulose | 47.810 |
| HPMC (e.g. Pharmacoat 606) | 2.500 |
| Carboxymethylcellulose sodium | 5.000 |
| Magnesium stearate | 1.250 |

### Coating

| **Constituents** | **mg/ tablet** |
|---|---|
| HPMC (e.g. Pharmacoat 606) | 2.400 |
| Polyethylene Glycol 6000 | 0.700 |
| Titanium dioxide | 1.000 |
| Talc | 0.857 |
| Iron oxide red | 0.043 |
| | |
| **Total Film coated tablet** | **255.000** |

### I) Film coated tablet

### Core

| **Constituents** | **mg/tablet** |
|---|---|
| Flibanserin | 100.000 |
| Lactose monohydrate | 171.080 |
| Microcrystalline cellulose | 57.020 |
| HPMC (e.g. Methocel E5) | 3.400 |
| Carboxymethylcellulose sodium | 6.800 |
| Magnesium stearate | 1.700 |

### Coating

| **Constituents** | **mg/ tablet** |
|---|---|
| HPMC (e.g. Methocel E5) | 3.360 |
| Polyethylene Glycol 6000 | 0.980 |
| Titanium dioxide | 1.400 |
| Talc | 1.200 |
| Iron oxide red | 0.060 |
| | |
| **Total Film coated tablet** | **347.000** |

### J) Film coated tablet

### Core

| **Constituents** | **mg/tablet** |
|---|---|
| Flibanserin | 2.000 |
| Dibasic Calciumphosphate, anhydrous | 61.010 |
| Microcrystalline cellulose | 61.010 |
| HPMC (Methocel E5) | 1.950 |
| Carboxymethylcellulose sodium | 2:600 |
| Colloidal silicon dioxide | 0.650 |
| Magnesium stearate | 0.780 |

### Coating

| **Constituents** | **mg/ tablet** |
|---|---|
| HPMC (Methocel E5) | 1.440 |
| Polyethylene Glycol 6000 | 0.420 |
| Titanium dioxide | 0.600 |
| Talc | 0.514 |
| Iron oxide red | 0.026 |
| | |
| **Total Film coated tablet** | **133.000** |

### K) Film coated tablet

### Core

| **Constituents** | **mg/tablet** |
|---|---|
| Flibanserin | 100.000 |
| Dibasic Calciumphosphate, anhydrous | 69.750 |
| Microcrystalline cellulose | 69.750 |
| HPMC (e.g. Methocel E5) | 2.750 |
| Carboxymethylcellulose sodium | 5.000 |
| Colloidal silicon dioxide | 1.250 |
| Magnesium stearate | 1.500 |

### Coating

| **Constituents** | **mg/tablet** |
|---|---|
| HPMC (e.g. Methocel E5) | 2.400 |
| Polyethylene Glycol 6000 | 0.700 |
| Titanium dioxide | 1.043 |
| Talc | 0.857 |
| | |
| **Total Film coated tablet** | **255.000** |

### L) Film coated tablet

### Core

| **Constituents** | **mg/tablet** |
|---|---|
| Flibanserin | 20.000 |
| Lactose monohydrate | 130.000 |
| Microcrystalline cellulose | 43.100 |
| Hydroxypropyl Cellulose (e.g. Klucel LF) | 1.900 |
| Sodium Starch Glycolate | 4.000 |
| Magnesium stearate | 1.000 |

### Coating

| **Constituents** | **mg/ tablet** |
|---|---|
| HPMC (e.g. Methocel E5) | 2.400 |
| Polyethylene Glycol 6000 | 0.700 |
| Titanium dioxide | 1.043 |
| Talc | 0.857 |
| | |
| **Total Film coated tablet** | **205.000** |

## Claims

1. Use of a therapeutically effective amount of flibanserin, optionally in form of the pharmacologically acceptable acid addition salts thereof, for the manufacture of a medicament for the treatment of premenstrual disorders.

2. Use according to claim 1 for the treatment of premenstrual disorders selected from the group consisting of premenstrual dysphoria, premenstrual syndrome and premenstrual dysphoric disorder.

3. Use according to claim 1 or 2, **characterized in that** flibanserin is applied in form of a pharmaceutically acceptable acid addition salt selected from the salts formed by the acids selected from, succinic acid, hydrobromic acid, acetic acid, fumaric acid, maleic acid, methanesulphonic acid, lactic acid, phosphoric acid, hydrochloric acid, sulphuric acid, tartaric acid, citric acid, and mixtures thereof.

4. Use according to one or more of claims 1 to 3, **characterized in that** flibanserin is applied in a dosis range between 0.1 to 400 mg per day.

## Patentansprüche

1. Verwendung einer therapeutisch wirksamen Menge von Flibanserin, gegebenenfalls in Form der pharmakologisch akzeptablen Säureadditionssalze davon, zur Herstellung eines Arzneimittels zur Behandlung prämenstrueller Störungen.

2. Verwendung nach Anspruch 1 zur Behandlung prämenstrueller Störungen, ausgewählt aus der Gruppe, bestehend aus prämenstrueller Dysphorie, prämenstruellem Syndrom und prämenstrueller dysphorischer Störung.

3. Verwendung nach Anspruch 1 oder 2, **dadurch gekennzeichnet, dass** Flibanserin in Form eines pharmazeutisch akzeptablen Säureadditionssalzes angewendet wird, ausgewählt aus den Salzen, gebildet aus den Säuren, ausgewählt aus Bernsteinsäure, Bromwasserstoffsäure, Essigsäure, Fumarsäure, Maleinsäure, Methansulfonsäure, Milchsäure, Phosphorsäure, Salzsäure, Schwefelsäure, Weinsäure, Zitronensäure und Mischungen hiervon.

4. Verwendung nach einem oder mehreren der Ansprüche 1 bis 3, **dadurch gekennzeichnet, dass** Flibanserin in einem Dosisbereich zwischen 0,1 bis 400 mg pro Tag angewendet wird.

## Revendications

1. Utilisation d'une quantité thérapeutiquement efficace de flibansérine, éventuellement sous forme de ses sels d'addition d'acide pharmacologiquement acceptables, pour la fabrication d'un médicament pour le traitement des troubles prémenstruels.

2. Utilisation selon la revendication 1 pour le traitement des troubles prémenstruels choisis dans le groupe consistant en la dysphorie prémenstruelle, le syndrome prémenstruel et le trouble dysphorique prémenstruel.

3. Utilisation selon la revendication 1 ou 2 **caractérisée en ce que** la flibansérine est appliquée sous forme d'un sel d'addition d'acide pharmaceutiquement acceptable choisi parmi les sels formés par les acides choisis parmi l'acide succinique, l'acide bromhydrique, l'acide acétique, l'acide fumarique, l'acide maléique, l'acide méthanesulfonique, l'acide lactique, l'acide phosphorique, l'acide chlorhydrique, l'acide sulfurique, l'acide tartrique, l'acide citrique et leurs mélanges.

4. Utilisation selon une ou plusieurs des revendications 1 à 3 **caractérisée en ce que** la flibansérine est appliquée dans une plage de doses entre 0,1 et 400 mg par jour.
